# EUROPEAN PATENT APPLICATION

(11) **EP 4 144 866 A1**
(43) Date of publication of application: **08.03.2023**
(21) Application number: 21194895.5
(22) Date of filing: 03.09.2021
(51) Int. Cl.: C12Q 1/6888

(54) **METHOD FOR THE IDENTIFICATION AND TRACEABILITY OF ANIMAL PRODUCTS**

(71) Applicant: Cebal - Centro De Biotecnologia Agrícola E Agro-Alimentar Do Alentejo, 7800-309 Beja (PT)
(72) Inventor: COSTA DO AMARAL RAMOS, ANTÓNIO MARCOS, 7800-309 BEJA (PT); USIÉ CHIMENOS, ANA ISABEL, 7800-309 BEJA (PT); BRANCO GASPAR, DANIEL FILIPE, 7800-309 BEJA (PT); PINHEIRO RIBEIRO DE MEIRELES, BRÍGIDA DA NATIVIDADE, 2685-019 SACAVÉM (PT); CARVALHO MAGALHÃES, HUGO, 4700-284 BRAGA (PT); SANTOS BARBOSA, PEDRO, 1000-127 LISBOA (PT); FIALHO LEÃO, CÉLIA CRISTINA, 2780-157 OEIRAS (PT)
(74) Representative: Neves, Ana

(57) **Abstract**

The present patent application discloses a method for the genetic identification and traceability of animal products originating from the Alentejano Pig, Bisaro and Malhado de Alcobaça breeds. Said identification is determined based on a group of Single Nucleotide Polymorphisms (SNPs) herein significantly associated with a specific animal breed.

## Description

### Technical field

The present patent application relates to a method for the genetic identification and traceability of animal products originating from the Alentejano Pig, Bísaro and Malhado de Alcobaça breeds.

### Background art

In Portugal, several animal products are based on the use of a single breed, usually an autochthonous breed, which is usually one of the prerequisites for the award of a Protected Designation of Origin/Protected Geographical Indication (PDO/PGI). The high prices achieved by PDO/PGI products open the possibility for fraudulent products, which do not meet the specified standards for their production, to be introduced into the market. Therefore, a system capable of detecting these frauds and increasing consumer confidence in this type of product is needed, effectively ensuring the traceability of each product back to its breed of origin. The increased availability of sequencing data substantially increases the identification of breed-specific SNPs and variants. Previously, the use of specific SNPs in tests to assign individuals, of unknown origin, to the breeds of domestic animals from which they originated, was shown to be a very effective and safe tool. In the case of Alentejo pig breed, a system of this kind is necessary to ensure the molecular traceability of products derived from this breed, comparing it with other Portuguese breeds (Bísaro and Malhado de Alcobaça).

### Summary

The present patent application relates to a method for the genetic identification and traceability of animal products originating from the Alentejano Pig, Bísaro and Malhado de Alcobaça breeds.

The method for the genetic identification and traceability of animal products originating from the Alentejano Pig, Bísaro and Malhado de Alcobaça breeds comprises the following steps:
Preparation of an animal product biological sample and extraction of DNA from said sample;
Genotyping the DNA sample for the following Single Nucleotide Polymorphisms (SNPs),

| Chromosome | Position | Breed | Ref. AF | Sp. AF | Gene ID |
|---|---|---|---|---|---|
| 8 | 30 463 786 | Malhado de Alcobaça | 0 | 1 | ENSSSCG00000008789 |
| 8 | 31 473 380 | Malhado de Alcobaça | 0 | 1 | ENSSSCG00000042573 |
| 8 | 31 515 280 | Malhado de Alcobaça | 0 | 1 | ENSSSCG00000008796 |
| 8 | 31 523 240 | Malhado de Alcobaça | 0 | 1 | ENSSSCG00000008796 |
| 8 | 31 876 853 | Malhado de Alcobaça | 0 | 1 | ENSSSCG00000024496 |
| 8 | 39 676 467 | Malhado de Alcobaça | 0 | 1 | ENSSSCG00000023858 |
| 8 | 39 917 210 | Malhado de Alcobaça | 0 | 1 | ENSSSCG00000008836 |
| 8 | 40 958 169 | Malhado de Alcobaça | 0 | 1 | ENSSSCG00000008840 |
| 8 | 42 672 627 | Malhado de Alcobaça | 0 | 1 | ENSSSCG00000042356 |
| 8 | 74 453 833 | Malhado de Alcobaça | 0 | 1 | ENSSSCG00000008995 |
| 8 | 75 170 705 | Malhado de Alcobaça | 0 | 1 | ENSSSCG00000047056 |
| 9 | 105 519 488 | Malhado de Alcobaça | 0 | 1 | ENSSSCG00000015432 |
| 11 | 6 278 374 | Malhado de Alcobaça | 0 | 1 | ENSSSCG00000025996 |
| 13 | 68 477 880 | Bisaro | 0,071 | 0,929 | ENSSSCG00000011580 |
| 9 | 50 091 126 | Malhado de Alcobaça | 0,083 | 0,917 | ENSSSCG00000022961 |
| 9 | 50 856 225 | Malhado de Alcobaça | 0,083 | 0,917 | ENSSSCG00000049068 |
| 9 | 61 927 011 | Malhado de Alcobaça | 0,083 | 0,917 | ENSSSCG00000051307 |
| 14 | 138 562 706 | Malhado de Alcobaça | 0,083 | 0,917 | ENSSSCG00000010756 |
| 9 | 55 020 112 | Malhado de Alcobaça | 0,1 | 0,9 | ENSSSCG00000035237 |
| Y | 1 734 671 | Malhado de Alcobaça | 0,125 | 0,875 | ENSSSCG00000032439 |
| 1 | 94 219 477 | Malhado de Alcobaça | 0,167 | 0,833 | ENSSSCG00000041974 |
| 1 | 132 657 060 | Malhado de Alcobaça | 0,167 | 0,833 | ENSSSCG00000043529 |
| 2 | 12 123 408 | Malhado de Alcobaça | 0,167 | 0,833 | ENSSSCG00000040765 |
| 4 | 124 289 757 | Malhado de Alcobaça | 0,167 | 0,833 | ENSSSCG00000006900 |
| 7 | 121 144 429 | Malhado de Alcobaça | 0,167 | 0,833 | ENSSSCG00000037558 |
| 7 | 121 228 597 | Malhado de Alcobaça | 0,167 | 0,833 | ENSSSCG00000002516 |
| 8 | 45 949 062 | Malhado de Alcobaça | 0,167 | 0,833 | ENSSSCG00000032 722 |
| 8 | 45 979 289 | Malhado de Alcobaça | 0,167 | 0,833 | ENSSSCG00000032722 |
| 8 | 47 006 668 | Malhado de Alcobaça | 0,167 | 0,833 | ENSSSCG00000039268 |
| 8 | 47 011 168 | Malhado de Alcobaça | 0,167 | 0,833 | ENSSSCG00000008873 |
| 9 | 49 943 302 | Malhado de Alcobaça | 0,167 | 0,833 | ENSSSCG00000015139 |
| 9 | 50 008 637 | Malhado de Alcobaça | 0,167 | 0,833 | ENSSSCG00000022961 |
| 9 | 50 035 316 | Malhado de Alcobaça | 0,167 | 0,833 | ENSSSCG00000022961 |
| 9 | 50 057 592 | Malhado de Alcobaça | 0,167 | 0,833 | ENSSSCG00000022961 |
| 9 | 80 949 194 | Malhado de Alcobaça | 0,167 | 0,833 | ENSSSCG00000047616 |
| 9 | 80 986 407 | Malhado de Alcobaça | 0,167 | 0,833 | ENSSSCG00000047616 |
| 9 | 85 362 136 | Malhado de Alcobaça | 0,167 | 0,833 | ENSSSCG00000048170 |
| 10 | 62 421 524 | Malhado de Alcobaça | 0,167 | 0,833 | ENSSSCG00000043083 |
| 10 | 62 608 766 | Malhado de Alcobaça | 0,167 | 0,833 | ENSSSCG00000043929 |
| 10 | 62 817 742 | Malhado de Alcobaça | 0,167 | 0,833 | ENSSSCG00000043907 |
| 12 | 4 967 767 | Malhado de Alcobaça | 0,167 | 0,833 | ENSSSCG00000042738 |
| 12 | 5 082 683 | Malhado de Alcobaça | 0,167 | 0,833 | ENSSSCG00000029901 |
| 12 | 5 092 623 | Malhado de Alcobaça | 0,167 | 0,833 | ENSSSCG00000029901 |
| 12 | 5 414 534 | Malhado de Alcobaça | 0,167 | 0,833 | ENSSSCG00000017192 |
| 12 | 36 894 578 | Malhado de Alcobaça | 0,167 | 0,833 | ENSSSCG00000017676 |
| 17 | 46 490 348 | Malhado de Alcobaça | 0,167 | 0,833 | ENSSSCG00000022202 |
| 17 | 46 583 458 | Malhado de Alcobaça | 0,167 | 0,833 | ENSSSCG00000022202 |
| 17 | 46 784 050 | Malhado de Alcobaça | 0,167 | 0,833 | ENSSSCG00000007371 |
| 17 | 47 332 039 | Malhado de Alcobaça | 0,167 | 0,833 | ENSSSCG00000007382 |
| 8 | 37 745 914 | Bisaro | 0,214 | 0,786 | ENSSSCG00000008813 |
| 6 | 1 185 690 | Malhado de Alcobaça | 0,25 | 0,75 | ENSSSCG00000031875 |
| 6 | 1 222 388 | Malhado de Alcobaça | 0,25 | 0,75 | ENSSSCG00000031875 |
| 18 | 6 027 301 | Alentejano pig | 0,269 | 0,731 | ENSSSCG00000025737 |
| 3 | 7 705 183 | Bisaro | 0,286 | 0,714 | ENSSSCG00000031411 |
| 8 | 38 014 594 | Bisaro | 0,286 | 0,714 | ENSSSCG00000008819 |
| 13 | 37 667 | Bisaro | 0,286 | 0,714 | ENSSSCG00000046931 |
| X | 58 888 382 | Alentejano pig | 0,32 | 0,68 | ENSSSCG00000032167 |
| 3 | 7 687 030 | Alentejano pig | 0,327 | 0,673 | ENSSSCG00000031411 |
| 10 | 5 366 723 | Alentejano pig | 0,327 | 0,673 | ENSSSCG00000036162 |
| 13 | 81 873 217 | Alentejano pig | 0,327 | 0,673 | ENSSSCG00000034369 |
| 1 | 64 200 320 | Bisaro | 0,357 | 0,643 | ENSSSCG00000027002 |
| 1 | 64 205 977 | Bisaro | 0,357 | 0,643 | ENSSSCG00000027002 |
| 2 | 10 417 587 | Bisaro | 0,357 | 0,643 | ENSSSCG00000029938 |
| 2 | 10 434 296 | Bisaro | 0,357 | 0,643 | ENSSSCG00000013090 |
| 7 | 9 238 471 | Bisaro | 0,357 | 0,643 | ENSSSCG00000001052 |
| 7 | 9 391 091 | Bisaro | 0,357 | 0,643 | ENSSSCG00000001052 |
| 4 | 75 841 171 | Alentejano pig | 0,365 | 0,635 | ENSSSCG00000006250 |
| 5 | 5 095 200 | Alentejano pig | 0,385 | 0,615 | ENSSSCG00000027266 |
| 9 | 16 187 151 | Alentejano pig | 0,385 | 0,615 | ENSSSCG00000042094 |
| 9 | 63 496 114 | Bisaro | 0,417 | 0,583 | ENSSSCG00000046596 |
| 3 | 7 682 829 | Alentejano pig | 0,42 | 0,58 | ENSSSCG00000031411 |
| 1 | 62 238 376 | Bisaro | 0,429 | 0,571 | ENSSSCG00000050706 |
| 1 | 67 414 194 | Bisaro | 0,429 | 0,571 | ENSSSCG00000004358 |
| 1 | 67 429 240 | Bisaro | 0,429 | 0,571 | ENSSSCG00000004358 |
| 1 | 68 918 754 | Bisaro | 0,429 | 0,571 | ENSSSCG00000004361 |
| 1 | 205 617 574 | Bisaro | 0,429 | 0,571 | ENSSSCG00000005178 |
| 3 | 6 723 163 | Bisaro | 0,429 | 0,571 | ENSSSCG00000023320 |
| 4 | 108 525 549 | Bisaro | 0,429 | 0,571 | ENSSSCG00000026680 |
| 4 | 109 786 671 | Bisaro | 0,429 | 0,571 | ENSSSCG00000006809 |
| 6 | 38 833 545 | Bisaro | 0,429 | 0,571 | ENSSSCG00000044959 |
| 8 | 87 967 047 | Bisaro | 0,429 | 0,571 | ENSSSCG00000024596 |
| 9 | 20 492 696 | Bisaro | 0,429 | 0,571 | ENSSSCG00000014919 |
| 10 | 800 269 | Bisaro | 0,429 | 0,571 | ENSSSCG00000048127 |
| 10 | 833 466 | Bisaro | 0,429 | 0,571 | ENSSSCG00000043434 |
| 10 | 853 085 | Bisaro | 0,429 | 0,571 | ENSSSCG00000043434 |
| 10 | 1 274 850 | Bisaro | 0,429 | 0,571 | ENSSSCG00000030244 |
| 10 | 11 150 078 | Bisaro | 0,429 | 0,571 | ENSSSCG00000037997 |
| 13 | 161 730 897 | Bisaro | 0,429 | 0,571 | ENSSSCG00000037688 |
| 15 | 70 058 879 | Bisaro | 0,429 | 0,571 | ENSSSCG00000045707 |
| 17 | 37 269 043 | Bisaro | 0,429 | 0,571 | ENSSSCG00000021769 |
| 17 | 38 185 725 | Bisaro | 0,429 | 0,571 | ENSSSCG00000007284 |
| 17 | 62 025 449 | Bisaro | 0,429 | 0,571 | ENSSSCG00000038278 |
| 9 | 131 973 226 | Alentejano pig | 0,442 | 0,558 | ENSSSCG00000040650 |
| 18 | 16 915 846 | Alentejano pig | 0,462 | 0,538 | ENSSSCG00000021562 |
| 17 | 29 496 180 | Alentejano pig | 0,481 | 0,519 | ENSSSCG00000007112 |
| 18 | 31 146 752 | Alentejano pig | 0,481 | 0,519 | ENSSSCG00000039642 |
| 1 | 2 878 108 | Bisaro | 0,5 | 0,5 | ENSSSCG00000049704 |
| 1 | 7 041 985 | Bisaro | 0,5 | 0,5 | ENSSSCG00000004037 |
| 1 | 7 042 983 | Bisaro | 0,5 | 0,5 | ENSSSCG00000004037 |
| 1 | 42 477 515 | Bisaro | 0,5 | 0,5 | ENSSSCG00000044184 |
| 1 | 47 051 775 | Bisaro | 0,5 | 0,5 | ENSSSCG00000050391 |
| 1 | 60 329 718 | Bisaro | 0,5 | 0,5 | ENSSSCG00000048181 |
| 1 | 65 686 360 | Bisaro | 0,5 | 0,5 | ENSSSCG00000046887 |
| 1 | 65 733 960 | Bisaro | 0,5 | 0,5 | ENSSSCG00000046887 |
| 1 | 65 734 193 | Bisaro | 0,5 | 0,5 | ENSSSCG00000046887 |
| 1 | 65 903 810 | Bisaro | 0,5 | 0,5 | ENSSSCG00000004347 |
| 1 | 84 443 727 | Bisaro | 0,5 | 0,5 | ENSSSCG00000031482 |
| 1 | 90 423 307 | Bisaro | 0,5 | 0,5 | ENSSSCG00000004479 |
| 1 | 92 886 948 | Bisaro | 0,5 | 0,5 | ENSSSCG00000049718 |
| 1 | 92 976 586 | Bisaro | 0,5 | 0,5 | ENSSSCG00000033160 |
| 1 | 92 993 394 | Bisaro | 0,5 | 0,5 | ENSSSCG00000033160 |
| 1 | 93 006 347 | Bisaro | 0,5 | 0,5 | ENSSSCG00000045399 |
| 1 | 93 067 039 | Bisaro | 0,5 | 0,5 | ENSSSCG00000047499 |
| 2 | 17 180 956 | Alentejano pig | 0,5 | 0,5 | ENSSSCG00000013276 |
| 3 | 7 688 382 | Alentejano pig | 0,5 | 0,5 | ENSSSCG00000031411 |
| 3 | 7 718 307 | Alentejano pig | 0,5 | 0,5 | ENSSSCG00000051021 |
| 3 | 7 719 451 | Alentejano pig | 0,5 | 0,5 | ENSSSCG00000007639 |
| 3 | 7 719 456 | Alentejano pig | 0,5 | 0,5 | ENSSSCG00000007639 |
| 3 | 41 851 708 | Alentejano pig | 0,5 | 0,5 | ENSSSCG00000049347 |
| 4 | 57 041 855 | Alentejano pig | 0,5 | 0,5 | ENSSSCG00000031362 |
| 4 | 81 354 492 | Alentejano pig | 0,5 | 0,5 | ENSSSCG00000006287 |
| 4 | 81 354 502 | Alentejano pig | 0,5 | 0,5 | ENSSSCG00000006287 |
| 4 | 81 460 964 | Alentejano pig | 0,5 | 0,5 | ENSSSCG00000006289 |
| 4 | 81 461 101 | Alentejano pig | 0,5 | 0,5 | ENSSSCG00000006289 |
| 4 | 103 296 739 | Alentejano pig | 0,5 | 0,5 | ENSSSCG00000006730 |
| 7 | 40 624 711 | Alentejano pig | 0,5 | 0,5 | ENSSSCG00000001710 |
| 8 | 5 293 | Alentejano pig | 0,5 | 0,5 | ENSSSCG00000047999 |
| 13 | 44 930 | Alentejano pig | 0,5 | 0,5 | ENSSSCG00000046931 |
| 14 | 113 396 910 | Alentejano pig | 0,5 | 0,5 | ENSSSCG00000010579 |
| 1 | 223 298 138 | Alentejano pig | 0,519 | 0,481 | ENSSSCG00000035668 |
| 1 | 245 476 692 | Alentejano pig | 0,519 | 0,481 | ENSSSCG00000005403 |
| 2 | 133 251 508 | Alentejano pig | 0,519 | 0,481 | ENSSSCG00000048403 |
| 3 | 7 719 528 | Alentejano pig | 0,519 | 0,481 | ENSSSCG00000007639 |
| 3 | 7 739 025 | Alentejano pig | 0,519 | 0,481 | ENSSSCG00000046525 |
| 3 | 59 067 555 | Alentejano pig | 0,519 | 0,481 | ENSSSCG00000008228 |
| 4 | 54 771 671 | Alentejano pig | 0,519 | 0,481 | ENSSSCG00000051032 |
| 5 | 39 920 848 | Alentejano pig | 0,519 | 0,481 | ENSSSCG00000027503 |
| 5 | 40 053 513 | Alentejano pig | 0,519 | 0,481 | ENSSSCG00000027503 |
| 5 | 40 063 145 | Alentejano pig | 0,519 | 0,481 | ENSSSCG00000027503 |
| 6 | 77 051 136 | Alentejano pig | 0,519 | 0,481 | ENSSSCG00000023732 |
| 6 | 168 227 970 | Alentejano pig | 0,519 | 0,481 | ENSSSCG00000003960 |
| 8 | 5 386 | Alentejano pig | 0,519 | 0,481 | ENSSSCG00000047999 |
| 8 | 26 104 721 | Alentejano pig | 0,519 | 0,481 | ENSSSCG00000008767 |
| 13 | 177 038 091 | Alentejano pig | 0,519 | 0,481 | ENSSSCG00000048010 |
| 1 | 93 145 928 | Alentejano pig | 0,538 | 0,462 | ENSSSCG00000031966 |
| 1 | 129 366 035 | Alentejano pig | 0,538 | 0,462 | ENSSSCG00000037292 |
| 1 | 157 286 063 | Alentejano pig | 0,538 | 0,462 | ENSSSCG00000045840 |
| 3 | 7 685 456 | Alentejano pig | 0,538 | 0,462 | ENSSSCG00000031411 |
| 3 | 7 717 958 | Alentejano pig | 0,538 | 0,462 | ENSSSCG00000051021 |
| 3 | 7 718 387 | Alentejano pig | 0,538 | 0,462 | ENSSSCG00000051021 |

Determining the animal product's origin and breed based on the presence of at least one of said SNPs significantly associated with a specific animal breed.

In one embodiment, the sample analyzed is preferably a blood sample.

### Detailed Description

The present patent application discloses a method for the genetic identification and traceability of animal products originating from the Alentejano Pig, Bísaro and Malhado de Alcobaça breeds. Genetic identification is determined based on a group of Single Nucleotide Polymorphisms (SNPs) disclosed herein which are associated with the pig breeds of Alentejano Pig, Bísaro and Malhado de Alcobaça.

The technology disclosed herein is based on the sequencing of genomes of animals of Alentejano Pig, Bísaro and Malhado de Alcobaça pig breeds, to identify the global set of variations, namely SNPs, present in the genome of each animal. First, a genome sequencing step was performed for the identification, which yielded a large volume of SNPs (in the order of millions of SNPs). Next, this set of variants was analyzed for the presence of breed-specific variations. In this context, a SNP is considered specific when it presents an allele found only in that race, and in none of the other races analyzed. This set of SNPs can then be used to genetically distinguish animals from each of the breeds, when compared to the others. Additionally, this technology is extensible to Protected Designation of Origin (PDO) products produced from the Alentejano and Bísaro pig breeds, which require the exclusive use of pure breed animals. The traceability system can then be used to determine whether a given PDO product complies with the specifications required by the PDO specifications, in particular the requirement to come from a pure-bred animal.

In order to provide the method disclosed herein, the genomes of animals registered in the studbook of each breed were sequenced and analyzed in order to ensure the racial purity. The set of SNPs resulting from the genome sequencing was then analyzed to identify breed-specific SNPs. A SNP is considered specific when it presents an allele that is present in a given breed, but not in any of the other breeds analyzed. The number of breed-specific SNPs found was 182 for the Alentejo Pig, 939 for the Bisaro, and 549 for the Malhado de Alcobaça. The list with the 150 most breed-specific SNPs is found in table 1. The combination of this set of SNPs is the basis of a test to attribute any animal to the breed of origin, if this is the case, and it can also be used to rule out the hypothesis that this animal belongs to any of the breeds. The specificity of each SNP is the principle underlying the application of this method, which can also be used to confirm or rule out the authenticity of a PDO product originating from one of the included breeds.

**Table 1 - 150 breed-specific SNPs .**

| Chromosome | Position | Breed | Ref. AF | Sp. AF | Gene ID | Gen. loc |
|---|---|---|---|---|---|---|
| 8 | 30 463 786 | Malhado de Alcobaça | 0 | 1 | ENSSSCG00000008789 | upstream |
| 8 | 31 473 380 | Malhado de Alcobaça | 0 | 1 | ENSSSCG00000042573 | intergenic |
| 8 | 31 515 280 | Malhado de Alcobaça | 0 | 1 | ENSSSCG00000008796 | intronic |
| 8 | 31 523 240 | Malhado de Alcobaça | 0 | 1 | ENSSSCG00000008796 | intronic |
| 8 | 31 876 853 | Malhado de Alcobaça | 0 | 1 | ENSSSCG00000024496 | intronic |
| 8 | 39 676 467 | Malhado de Alcobaça | 0 | 1 | ENSSSCG00000023858 | intergenic |
| 8 | 39 917 210 | Malhado de Alcobaça | 0 | 1 | ENSSSCG00000 008836 | intronic |
| 8 | 40 958 169 | Malhado de Alcobaça | 0 | 1 | ENSSSCG00000008840 | intergenic |
| 8 | 42 672 627 | Malhado de Alcobaça | 0 | 1 | ENSSSCG00000042356 | intergenic |
| 8 | 74 453 833 | Malhado de Alcobaça | 0 | 1 | ENSSSCG00000008995 | intergenic |
| 8 | 75 170 705 | Malhado de Alcobaça | 0 | 1 | ENSSSCG00000047056 | intergenic |
| 9 | 105 519 488 | Malhado de Alcobaça | 0 | 1 | ENSSSCG00000015432 | intronic |
| 11 | 6 278 374 | Malhado de Alcobaça | 0 | 1 | ENSSSCG00000025996 | intronic |
| 13 | 68 477 880 | Bisaro | 0,071 | 0,929 | ENSSSCG00000011580 | intronic |
| 9 | 50 091 126 | Malhado de Alcobaça | 0,083 | 0,917 | ENSSSCG00000022961 | intronic |
| 9 | 50 856 225 | Malhado de Alcobaça | 0,083 | 0,917 | ENSSSCG00000049068 | intergenic |
| 9 | 61 927 011 | Malhado de Alcobaça | 0,083 | 0,917 | ENSSSCG00000051307 | intergenic |
| 14 | 138 562 706 | Malhado de Alcobaça | 0,083 | 0,917 | ENSSSCG00000010756 | intronic |
| 9 | 55 020 112 | Malhado de Alcobaça | 0,1 | 0,9 | ENSSSCG00000035237 | ncRNA_intr onic |
| Y | 1 734 671 | Malhado de Alcobaça | 0,125 | 0,875 | ENSSSCG00000032439 | intronic |
| 1 | 94 219 477 | Malhado de Alcobaça | 0,167 | 0,833 | ENSSSCG00000041974 | intergenic |
| 1 | 132 657 060 | Malhado de Alcobaça | 0,167 | 0,833 | ENSSSCG00000043529 | intergenic |
| 2 | 12 123 408 | Malhado de Alcobaça | 0,167 | 0,833 | ENSSSCG00000040765 | exonic |
| 4 | 124 289 757 | Malhado de Alcobaça | 0,167 | 0,833 | ENSSSCG00000006900 | intronic |
| 7 | 121 144 429 | Malhado de Alcobaça | 0,167 | 0,833 | ENSSSCG00000037558 | intronic |
| 7 | 121 228 597 | Malhado de Alcobaça | 0,167 | 0,833 | ENSSSCG00000002516 | intronic |
| 8 | 45 949 062 | Malhado de Alcobaça | 0,167 | 0,833 | ENSSSCG00000032722 | intronic |
| 8 | 45 979 289 | Malhado de Alcobaça | 0,167 | 0,833 | ENSSSCG00000032722 | intronic |
| 8 | 47 006 668 | Malhado de Alcobaça | 0,167 | 0,833 | ENSSSCG00000039268 | intergenic |
| 8 | 47 011 168 | Malhado de Alcobaça | 0,167 | 0,833 | ENSSSCG00000008873 | intronic |
| 9 | 49 943 302 | Malhado de Alcobaça | 0,167 | 0,833 | ENSSSCG00000015139 | intergenic |
| 9 | 50 008 637 | Malhado de Alcobaça | 0,167 | 0,833 | ENSSSCG00000022961 | intronic |
| 9 | 50 035 316 | Malhado de Alcobaça | 0,167 | 0,833 | ENSSSCG00000022961 | intronic |
| 9 | 50 057 592 | Malhado de Alcobaça | 0,167 | 0,833 | ENSSSCG00000022961 | intronic |
| 9 | 80 949 194 | Malhado de Alcobaça | 0,167 | 0,833 | ENSSSCG00000047616 | intergenic |
| 9 | 80 986 407 | Malhado de Alcobaça | 0,167 | 0,833 | ENSSSCG00000047616 | intergenic |
| 9 | 85 362 136 | Malhado de Alcobaça | 0,167 | 0,833 | ENSSSCG00000048170 | intergenic |
| 10 | 62 421 524 | Malhado de Alcobaça | 0,167 | 0,833 | ENSSSCG00000043083 | ncRNA_intr onic |
| 10 | 62 608 766 | Malhado de Alcobaça | 0,167 | 0,833 | ENSSSCG00000043929 | intergenic |
| 10 | 62 817 742 | Malhado de Alcobaça | 0,167 | 0,833 | ENSSSCG00000043907 | ncRNA_intr onic |
| 12 | 4 967 767 | Malhado de Alcobaça | 0,167 | 0,833 | ENSSSCG00000042738 | intergenic |
| 12 | 5 082 683 | Malhado de Alcobaça | 0,167 | 0,833 | ENSSSCG00000029901 | intronic |
| 12 | 5 092 623 | Malhado de Alcobaça | 0,167 | 0,833 | ENSSSCG00000029901 | intronic |
| 12 | 5 414 534 | Malhado de Alcobaça | 0,167 | 0,833 | ENSSSCG00000017192 | intronic |
| 12 | 36 894 578 | Malhado de Alcobaça | 0,167 | 0,833 | ENSSSCG00000017676 | downstream |
| 17 | 46 490 348 | Malhado de Alcobaça | 0,167 | 0,833 | ENSSSCG00000022202 | intronic |
| 17 | 46 583 458 | Malhado de Alcobaça | 0,167 | 0,833 | ENSSSCG00000022202 | intergenic |
| 17 | 46 784 050 | Malhado de Alcobaça | 0,167 | 0,833 | ENSSSCG00000007371 | intronic |
| 17 | 47 332 039 | Malhado de Alcobaça | 0,167 | 0,833 | ENSSSCG00000007382 | intronic |
| 8 | 37 745 914 | Bisaro | 0,214 | 0,786 | ENSSSCG00000008813 | intronic |
| 6 | 1 185 690 | Malhado de Alcobaça | 0,25 | 0,75 | ENSSSCG00000031875 | intergenic |
| 6 | 1 222 388 | Malhado de Alcobaça | 0,25 | 0,75 | ENSSSCG00000031875 | intergenic |
| 18 | 6 027 301 | Alentejano pig | 0,269 | 0,731 | ENSSSCG00000025737 | intronic |
| 3 | 7 705 183 | Bisaro | 0,286 | 0,714 | ENSSSCG00000031411 | intergenic |
| 8 | 38 014 594 | Bisaro | 0,286 | 0,714 | ENSSSCG00000008819 | intronic |
| 13 | 37 667 | Bisaro | 0,286 | 0,714 | ENSSSCG00000046931 | intergenic |
| X | 58 888 382 | Alentejano pig | 0,32 | 0,68 | ENSSSCG00000032167 | intergenic |
| 3 | 7 687 030 | Alentejano pig | 0,327 | 0,673 | ENSSSCG00000031411 | intergenic |
| 10 | 5 366 723 | Alentejano pig | 0,327 | 0,673 | ENSSSCG00000036162 | intergenic |
| 13 | 81 873 217 | Alentejano pig | 0,327 | 0,673 | ENSSSCG00000034369 | intronic |
| 1 | 64 200 320 | Bisaro | 0,357 | 0,643 | ENSSSCG00000027002 | intronic |
| 1 | 64 205 977 | Bisaro | 0,357 | 0,643 | ENSSSCG00000027002 | intronic |
| 2 | 10 417 587 | Bisaro | 0,357 | 0,643 | ENSSSCG00000029938 | intronic |
| 2 | 10 434 296 | Bisaro | 0,357 | 0,643 | ENSSSCG00000013090 | intronic |
| 7 | 9 238 471 | Bisaro | 0,357 | 0,643 | ENSSSCG00000001052 | intronic |
| 7 | 9 391 091 | Bisaro | 0,357 | 0,643 | ENSSSCG00000001052 | intronic |
| 4 | 75 841 171 | Alentejano pig | 0,365 | 0,635 | ENSSSCG00000006250 | intronic |
| 5 | 5 095 200 | Alentejano pig | 0,385 | 0,615 | ENSSSCG00000027266 | intergenic |
| 9 | 16 187 151 | Alentejano pig | 0,385 | 0,615 | ENSSSCG00000042094 | intergenic |
| 9 | 63 496 114 | Bisaro | 0,417 | 0,583 | ENSSSCG00000046596 | intergenic |
| 3 | 7 682 829 | Alentejano pig | 0,42 | 0,58 | ENSSSCG00000031411 | intergenic |
| 1 | 62 238 376 | Bisaro | 0,429 | 0,571 | ENSSSCG00000050706 | intergenic |
| 1 | 67 414 194 | Bisaro | 0,429 | 0,571 | ENSSSCG00000004358 | intronic |
| 1 | 67 429 240 | Bisaro | 0,429 | 0,571 | ENSSSCG00000004358 | intronic |
| 1 | 68 918 754 | Bisaro | 0,429 | 0,571 | ENSSSCG00000004361 | intergenic |
| 1 | 205 617 574 | Bisaro | 0,429 | 0,571 | ENSSSCG00000005178 | intronic |
| 3 | 6 723 163 | Bisaro | 0,429 | 0,571 | ENSSSCG00000023320 | intronic |
| 4 | 108 525 549 | Bisaro | 0,429 | 0,571 | ENSSSCG00000026680 | intronic |
| 4 | 109 786 671 | Bisaro | 0,429 | 0,571 | ENSSSCG00000006809 | upstream |
| 6 | 38 833 545 | Bisaro | 0,429 | 0,571 | ENSSSCG00000044959 | intergenic |
| 8 | 87 967 047 | Bisaro | 0,429 | 0,571 | ENSSSCG00000024596 | intergenic |
| 9 | 20 492 696 | Bisaro | 0,429 | 0,571 | ENSSSCG00000014919 | intronic |
| 10 | 800 269 | Bisaro | 0,429 | 0,571 | ENSSSCG00000048127 | intergenic |
| 10 | 833 466 | Bisaro | 0,429 | 0,571 | ENSSSCG00000043434 | intergenic |
| 10 | 853 085 | Bisaro | 0,429 | 0,571 | ENSSSCG00000043434 | intergenic |
| 10 | 1 274 850 | Bisaro | 0,429 | 0,571 | ENSSSCG00000030244 | intergenic |
| 10 | 11 150 078 | Bisaro | 0,429 | 0,571 | ENSSSCG00000037997 | ncRNA_intr onic |
| 13 | 161 730 897 | Bisaro | 0,429 | 0,571 | ENSSSCG00000037688 | intergenic |
| 15 | 70 058 879 | Bisaro | 0,429 | 0,571 | ENSSSCG00000045707 | exonic |
| 17 | 37 269 043 | Bisaro | 0,429 | 0,571 | ENSSSCG00000021769 | intronic |
| 17 | 38 185 725 | Bisaro | 0,429 | 0,571 | ENSSSCG00000007284 | intronic |
| 17 | 62 025 449 | Bisaro | 0,429 | 0,571 | ENSSSCG00000038278 | intronic |
| 9 | 131 97 3 226 | Alentejano pig | 0,442 | 0,558 | ENSSSCG00000040650 | intronic |
| 18 | 16 915 846 | Alentejano pig | 0,462 | 0,538 | ENSSSCG00000021562 | intronic |
| 17 | 29 496 180 | Alentejano pig | 0,481 | 0,519 | ENSSSCG00000007112 | intergenic |
| 18 | 31 146 752 | Alentejano pig | 0,481 | 0,519 | ENSSSCG00000039642 | intergenic |
| 1 | 2 878 108 | Bisaro | 0,5 | 0,5 | ENSSSCG00000049704 | intergenic |
| 1 | 7 041 985 | Bisaro | 0,5 | 0,5 | ENSSSCG00000004037 | intronic |
| 1 | 7 042 983 | Bisaro | 0,5 | 0,5 | ENSSSCG00000004037 | intronic |
| 1 | 42 477 515 | Bisaro | 0,5 | 0,5 | ENSSSCG00000044184 | intergenic |
| 1 | 47 051 775 | Bisaro | 0,5 | 0,5 | ENSSSCG00000050391 | intergenic |
| 1 | 60 329 718 | Bisaro | 0,5 | 0,5 | ENSSSCG00000048181 | intergenic |
| 1 | 65 686 360 | Bisaro | 0,5 | 0,5 | ENSSSCG00000046887 | intergenic |
| 1 | 65 733 960 | Bisaro | 0,5 | 0,5 | ENSSSCG00000046887 | intergenic |
| 1 | 65 734 193 | Bisaro | 0,5 | 0,5 | ENSSSCG00000046887 | intergenic |
| 1 | 65 903 810 | Bisaro | 0,5 | 0,5 | ENSSSCG00000004347 | intronic |
| 1 | 84 443 727 | Bisaro | 0,5 | 0,5 | ENSSSCG00000031482 | intergenic |
| 1 | 90 423 307 | Bisaro | 0,5 | 0,5 | ENSSSCG00000004479 | intronic |
| 1 | 92 886 948 | Bisaro | 0,5 | 0,5 | ENSSSCG00000049718 | intergenic |
| 1 | 92 976 586 | Bisaro | 0,5 | 0,5 | ENSSSCG00000033160 | intergenic |
| 1 | 92 993 394 | Bisaro | 0,5 | 0,5 | ENSSSCG00000033160 | intergenic |
| 1 | 93 006 347 | Bisaro | 0,5 | 0,5 | ENSSSCG00000045399 | downstream |
| 1 | 93 067 039 | Bisaro | 0,5 | 0,5 | ENSSSCG00000047499 | ncRNA_intr onic |
| 2 | 17 180 956 | Alentejano pig | 0,5 | 0,5 | ENSSSCG00000013276 | exonic |
| 3 | 7 688 382 | Alentejano pig | 0,5 | 0,5 | ENSSSCG00000031411 | intergenic |
| 3 | 7 718 307 | Alentejano pig | 0,5 | 0,5 | ENSSSCG00000051021 | intergenic |
| 3 | 7 719 451 | Alentejano pig | 0,5 | 0,5 | ENSSSCG00000007639 | downstream |
| 3 | 7 719 456 | Alentejano pig | 0,5 | 0,5 | ENSSSCG00000007639 | downstream |
| 3 | 41 851 708 | Alentejano pig | 0,5 | 0,5 | ENSSSCG00000049347 | intergenic |
| 4 | 57 041 855 | Alentejano pig | 0,5 | 0,5 | ENSSSCG00000031362 | intergenic |
| 4 | 81 354 492 | Alentejano pig | 0,5 | 0,5 | ENSSSCG00000006287 | intergenic |
| 4 | 81 354 502 | Alentejano pig | 0,5 | 0,5 | ENSSSCG00000006287 | intergenic |
| 4 | 81 460 964 | Alentejano pig | 0,5 | 0,5 | ENSSSCG00000006289 | intronic |
| 4 | 81 461 101 | Alentejano pig | 0,5 | 0,5 | ENSSSCG00000006289 | intronic |
| 4 | 103 296 739 | Alentejano pig | 0,5 | 0,5 | ENSSSCG00000006730 | intronic |
| 7 | 40 624 711 | Alentejano pig | 0,5 | 0,5 | ENSSSCG00000001710 | intergenic |
| 8 | 5 293 | Alentejano pig | 0,5 | 0,5 | ENSSSCG00000047999 | intergenic |
| 13 | 44 930 | Alentejano pig | 0,5 | 0,5 | ENSSSCG00000046931 | intergenic |
| 14 | 113 396 910 | Alentejano pig | 0,5 | 0,5 | ENSSSCG00000010579 | intergenic |
| 1 | 223 298 138 | Alentejano pig | 0,519 | 0,481 | ENSSSCG00000035668 | intergenic |
| 1 | 245 476 692 | Alentejano pig | 0,519 | 0,481 | ENSSSCG00000005403 | intronic |
| 2 | 133 251 508 | Alentejano pig | 0,519 | 0,481 | ENSSSCG00000048403 | intergenic |
| 3 | 7 719 528 | Alentejano pig | 0,519 | 0,481 | ENSSSCG00000007639 | downstream |
| 3 | 7 739 025 | Alentejano pig | 0,519 | 0,481 | ENSSSCG00000046525 | intergenic |
| 3 | 59 067 555 | Alentejano pig | 0,519 | 0,481 | ENSSSCG00000008228 | intronic |
| 4 | 54 771 671 | Alentejano pig | 0,519 | 0,481 | ENSSSCG00000051032 | intergenic |
| 5 | 39 920 848 | Alentejano pig | 0,519 | 0,481 | ENSSSCG00000027503 | intergenic |
| 5 | 40 053 513 | Alentejano pig | 0,519 | 0,481 | ENSSSCG00000027503 | intergenic |
| 5 | 40 063 145 | Alentejano pig | 0,519 | 0,481 | ENSSSCG00000027503 | intergenic |
| 6 | 77 051 136 | Alentejano pig | 0,519 | 0,481 | ENSSSCG00000023732 | intergenic |
| 6 | 168 227 970 | Alentejano pig | 0,519 | 0,481 | ENSSSCG00000003960 | intergenic |
| 8 | 5 386 | Alentejano pig | 0,519 | 0,481 | ENSSSCG00000047999 | intergenic |
| 8 | 26 104 721 | Alentejano pig | 0,519 | 0,481 | ENSSSCG00000008767 | intergenic |
| 13 | 177 038 091 | Alentejano pig | 0,519 | 0,481 | ENSSSCG00000048010 | intergenic |
| 1 | 93 145 928 | Alentejano pig | 0,538 | 0,462 | ENSSSCG00000031966 | intergenic |
| 1 | 129 366 035 | Alentejano pig | 0,538 | 0,462 | ENSSSCG00000037292 | intergenic |
| 1 | 157 286 063 | Alentejano pig | 0,538 | 0,462 | ENSSSCG00000045840 | intergenic |
| 3 | 7 685 456 | Alentejano pig | 0,538 | 0,462 | ENSSSCG00000031411 | intergenic |
| 3 | 7 717 958 | Alentejano pig | 0,538 | 0,462 | ENSSSCG00000051021 | intergenic |
| 3 | 7 718 387 | Alentejano pig | 0,538 | 0,462 | ENSSSCG00000051021 | intergenic |

The method of the present patent application involves several steps. Initially, it is necessary to collect a sample of biological material, such as a blood sample, for DNA extraction. Next, this DNA sample is genotyped for the SNPs of interest, and the genotypes obtained for each SNP are evaluated. The decision to determine the animal of origin is based on the presence of the genotype(s) significantly associated with a specific pig breed.

In one embodiment, the sample analyzed is preferably a blood sample.

## Claims

1. Method for the genetic identification and traceability of animal products originating from the Alentejano Pig, Bísaro and Malhado de Alcobaça breeds comprises the following steps:
Preparation of an animal product biological sample and extraction of DNA from said sample;
Genotyping the DNA sample for the following Single Nucleotide Polymorphisms (SNPs),
| Chromosome | Position | Breed | Ref. AF | Sp. AF | Gene ID |
|---|---|---|---|---|---|
| 8 | 30 463 786 | Malhado de Alcobaça | 0 | 1 | ENSSSCG00000008789 |
| 8 | 31 473 380 | Malhado de Alcobaça | 0 | 1 | ENSSSCG00000042573 |
| 8 | 31 515 280 | Malhado de Alcobaça | 0 | 1 | ENSSSCG00000008796 |
| 8 | 31 523 240 | Malhado de Alcobaça | 0 | 1 | ENSSSCG00000008796 |
| 8 | 31 876 853 | Malhado de Alcobaça | 0 | 1 | ENSSSCG00000024496 |
| 8 | 39 676 467 | Malhado de Alcobaça | 0 | 1 | ENSSSCG00000023858 |
| 8 | 39 917 210 | Malhado de Alcobaça | 0 | 1 | ENSSSCG00000008836 |
| 8 | 40 958 169 | Malhado de Alcobaça | 0 | 1 | ENSSSCG00000008840 |
| 8 | 42 672 627 | Malhado de Alcobaça | 0 | 1 | ENSSSCG00000042356 |
| 8 | 74 453 833 | Malhado de Alcobaça | 0 | 1 | ENSSSCG00000008995 |
| 8 | 75 170 705 | Malhado de Alcobaça | 0 | 1 | ENSSSCG00000047056 |
| 9 | 105 519 488 | Malhado de Alcobaça | 0 | 1 | ENSSSCG00000015432 |
| 11 | 6 278 374 | Malhado de Alcobaça | 0 | 1 | ENSSSCG00000025996 |
| 13 | 68 477 880 | Bisaro | 0,071 | 0,929 | ENSSSCG00000011580 |
| 9 | 50 091 126 | Malhado de Alcobaça | 0,083 | 0,917 | ENSSSCG00000022961 |
| 9 | 50 856 225 | Malhado de Alcobaça | 0,083 | 0,917 | ENSSSCG00000049068 |
| 9 | 61 927 011 | Malhado de Alcobaça | 0,083 | 0,917 | ENSSSCG00000051307 |
| 14 | 138 562 706 | Malhado de Alcobaça | 0,083 | 0,917 | ENSSSCG00000010756 |
| 9 | 55 020 112 | Malhado de Alcobaça | 0,1 | 0,9 | ENSSSCG00000035237 |
| Y | 1 734 671 | Malhado de Alcobaça | 0,125 | 0,875 | ENSSSCG00000032439 |
| 1 | 94 219 477 | Malhado de Alcobaça | 0,167 | 0,833 | ENSSSCG00000041974 |
| 1 | 132 657 060 | Malhado de Alcobaça | 0,167 | 0,833 | ENSSSCG00000043529 |
| 2 | 12 123 408 | Malhado de Alcobaça | 0,167 | 0,833 | ENSSSCG00000040765 |
| 4 | 124 289 757 | Malhado de Alcobaça | 0,167 | 0,833 | ENSSSCG00000006900 |
| 7 | 121 144 429 | Malhado de Alcobaça | 0,167 | 0,833 | ENSSSCG00000037558 |
| 7 | 121 228 597 | Malhado de Alcobaça | 0,167 | 0,833 | ENSSSCG00000002516 |
| 8 | 45 949 062 | Malhado de Alcobaça | 0,167 | 0,833 | ENSSSCG00000032722 |
| 8 | 45 979 289 | Malhado de Alcobaça | 0,167 | 0,833 | ENSSSCG00000032722 |
| 8 | 47 006 668 | Malhado de Alcobaça | 0,167 | 0,833 | ENSSSCG00000039268 |
| 8 | 47 011 168 | Malhado de Alcobaça | 0,167 | 0,833 | ENSSSCG00000008873 |
| 9 | 49 943 302 | Malhado de Alcobaça | 0,167 | 0,833 | ENSSSCG00000015139 |
| 9 | 50 008 637 | Malhado de Alcobaça | 0,167 | 0,833 | ENSSSCG00000022961 |
| 9 | 50 035 316 | Malhado de Alcobaça | 0,167 | 0,833 | ENSSSCG00000022961 |
| 9 | 50 057 592 | Malhado de Alcobaça | 0,167 | 0,833 | ENSSSCG00000022961 |
| 9 | 80 949 194 | Malhado de Alcobaça | 0,167 | 0,833 | ENSSSCG00000047616 |
| 9 | 80 986 407 | Malhado de Alcobaça | 0,167 | 0,833 | ENSSSCG00000047616 |
| 9 | 85 362 136 | Malhado de Alcobaça | 0,167 | 0,833 | ENSSSCG00000048170 |
| 10 | 62 421 524 | Malhado de Alcobaça | 0,167 | 0,833 | ENSSSCG00000043083 |
| 10 | 62 608 766 | Malhado de Alcobaça | 0,167 | 0,833 | ENSSSCG00000043929 |
| 10 | 62 817 742 | Malhado de Alcobaça | 0,167 | 0,833 | ENSSSCG00000043907 |
| 12 | 4 967 767 | Malhado de Alcobaça | 0,167 | 0,833 | ENSSSCG00000042738 |
| 12 | 5 082 683 | Malhado de Alcobaça | 0,167 | 0,833 | ENSSSCG00000029901 |
| 12 | 5 092 623 | Malhado de Alcobaça | 0,167 | 0,833 | ENSSSCG00000029901 |
| 12 | 5 414 534 | Malhado de Alcobaça | 0,167 | 0,833 | ENSSSCG00000017192 |
| 12 | 36 894 578 | Malhado de Alcobaça | 0,167 | 0,833 | ENSSSCG00000017676 |
| 17 | 46 490 348 | Malhado de Alcobaça | 0,167 | 0,833 | ENSSSCG00000022202 |
| 17 | 46 583 458 | Malhado de Alcobaça | 0,167 | 0,833 | ENSSSCG00000022202 |
| 17 | 46 784 050 | Malhado de Alcobaça | 0,167 | 0,833 | ENSSSCG00000007371 |
| 17 | 47 332 039 | Malhado de Alcobaça | 0,167 | 0,833 | ENSSSCG00000007382 |
| 8 | 37 745 914 | Bisaro | 0,214 | 0,786 | ENSSSCG00000008813 |
| 6 | 1 185 690 | Malhado de Alcobaça | 0,25 | 0,75 | ENSSSCG00000031875 |
| 6 | 1 222 388 | Malhado de Alcobaça | 0,25 | 0,75 | ENSSSCG00000031875 |
| 18 | 6 027 301 | Alentejano pig | 0,269 | 0,731 | ENSSSCG00000025737 |
| 3 | 7 705 183 | Bisaro | 0,286 | 0,714 | ENSSSCG00000031411 |
| 8 | 38 014 594 | Bisaro | 0,286 | 0,714 | ENSSSCG00000008819 |
| 13 | 37 667 | Bisaro | 0,286 | 0,714 | ENSSSCG00000046931 |
| X | 58 888 382 | Alentejano pig | 0,32 | 0,68 | ENSSSCG00000032167 |
| 3 | 7 687 030 | Alentejano pig | 0,327 | 0,673 | ENSSSCG00000031411 |
| 10 | 5 366 723 | Alentejano pig | 0,327 | 0,673 | ENSSSCG00000036162 |
| 13 | 81 873 217 | Alentejano pig | 0,327 | 0,673 | ENSSSCG00000034369 |
| 1 | 64 200 320 | Bisaro | 0,357 | 0,643 | ENSSSCG00000027002 |
| 1 | 64 205 977 | Bisaro | 0,357 | 0,643 | ENSSSCG00000027002 |
| 2 | 10 417 587 | Bisaro | 0,357 | 0,643 | ENSSSCG00000029938 |
| 2 | 10 434 296 | Bisaro | 0,357 | 0,643 | ENSSSCG00000013090 |
| 7 | 9 238 471 | Bisaro | 0,357 | 0,643 | ENSSSCG00000001052 |
| 7 | 9 391 091 | Bisaro | 0,357 | 0,643 | ENSSSCG00000001052 |
| 4 | 75 841 171 | Alentejano pig | 0,365 | 0,635 | ENSSSCG00000006250 |
| 5 | 5 095 200 | Alentejano pig | 0,385 | 0,615 | ENSSSCG00000027266 |
| 9 | 16 187 151 | Alentejano pig | 0,385 | 0,615 | ENSSSCG00000042094 |
| 9 | 63 496 114 | Bisaro | 0,417 | 0,583 | ENSSSCG00000046596 |
| 3 | 7 682 829 | Alentejano pig | 0,42 | 0,58 | ENSSSCG00000031411 |
| 1 | 62 238 376 | Bisaro | 0,429 | 0,571 | ENSSSCG00000050706 |
| 1 | 67 414 194 | Bisaro | 0,429 | 0,571 | ENSSSCG00000004358 |
| 1 | 67 429 240 | Bisaro | 0,429 | 0,571 | ENSSSCG00000004358 |
| 1 | 68 918 754 | Bisaro | 0,429 | 0,571 | ENSSSCG00000004361 |
| 1 | 205 617 574 | Bisaro | 0,429 | 0,571 | ENSSSCG00000005178 |
| 3 | 6 723 163 | Bisaro | 0,429 | 0,571 | ENSSSCG00000023320 |
| 4 | 108 525 549 | Bisaro | 0,429 | 0,571 | ENSSSCG00000026680 |
| 4 | 109 786 671 | Bisaro | 0,429 | 0,571 | ENSSSCG00000006809 |
| 6 | 38 833 545 | Bisaro | 0,429 | 0,571 | ENSSSCG00000044959 |
| 8 | 87 967 047 | Bisaro | 0,429 | 0,571 | ENSSSCG00000024596 |
| 9 | 20 492 696 | Bisaro | 0,429 | 0,571 | ENSSSCG00000014919 |
| 10 | 800 269 | Bisaro | 0,429 | 0,571 | ENSSSCG00000048127 |
| 10 | 833 466 | Bisaro | 0,429 | 0,571 | ENSSSCG00000043434 |
| 10 | 853 085 | Bisaro | 0,429 | 0,571 | ENSSSCG00000043434 |
| 10 | 1 274 850 | Bisaro | 0,429 | 0,571 | ENSSSCG00000030244 |
| 10 | 11 150 078 | Bisaro | 0,429 | 0,571 | ENSSSCG00000037997 |
| 13 | 161 730 897 | Bisaro | 0,429 | 0,571 | ENSSSCG00000037688 |
| 15 | 70 058 879 | Bisaro | 0,429 | 0,571 | ENSSSCG00000045 707 |
| 17 | 37 269 043 | Bisaro | 0,429 | 0,571 | ENSSSCG00000021769 |
| 17 | 38 185 725 | Bisaro | 0,429 | 0,571 | ENSSSCG00000007284 |
| 17 | 62 025 449 | Bisaro | 0,429 | 0,571 | ENSSSCG00000038278 |
| 9 | 131 973 226 | Alentejano pig | 0,442 | 0,558 | ENSSSCG00000040650 |
| 18 | 16 915 846 | Alentejano pig | 0,462 | 0,538 | ENSSSCG00000021562 |
| 17 | 29 496 180 | Alentejano pig | 0,481 | 0,519 | ENSSSCG00000007112 |
| 18 | 31 146 752 | Alentejano pig | 0,481 | 0,519 | ENSSSCG00000039642 |
| 1 | 2 878 108 | Bisaro | 0,5 | 0,5 | ENSSSCG00000049704 |
| 1 | 7 041 985 | Bisaro | 0,5 | 0,5 | ENSSSCG00000004037 |
| 1 | 7 042 983 | Bisaro | 0,5 | 0,5 | ENSSSCG00000004037 |
| 1 | 42 477 515 | Bisaro | 0,5 | 0,5 | ENSSSCG00000044184 |
| 1 | 47 051 775 | Bisaro | 0,5 | 0,5 | ENSSSCG00000050391 |
| 1 | 60 329 718 | Bisaro | 0,5 | 0,5 | ENSSSCG00000048181 |
| 1 | 65 686 360 | Bisaro | 0,5 | 0,5 | ENSSSCG00000046887 |
| 1 | 65 733 960 | Bisaro | 0,5 | 0,5 | ENSSSCG00000046887 |
| 1 | 65 734 193 | Bisaro | 0,5 | 0,5 | ENSSSCG00000046887 |
| 1 | 65 903 810 | Bisaro | 0,5 | 0,5 | ENSSSCG00000004347 |
| 1 | 84 443 727 | Bisaro | 0,5 | 0,5 | ENSSSCG00000031482 |
| 1 | 90 423 307 | Bisaro | 0,5 | 0,5 | ENSSSCG00000004479 |
| 1 | 92 886 948 | Bisaro | 0,5 | 0,5 | ENSSSCG00000049718 |
| 1 | 92 976 586 | Bisaro | 0,5 | 0,5 | ENSSSCG00000033160 |
| 1 | 92 993 394 | Bisaro | 0,5 | 0,5 | ENSSSCG00000033160 |
| 1 | 93 006 347 | Bisaro | 0,5 | 0,5 | ENSSSCG00000045399 |
| 1 | 93 067 039 | Bisaro | 0,5 | 0,5 | ENSSSCG00000047499 |
| 2 | 17 180 956 | Alentejano pig | 0,5 | 0,5 | ENSSSCG00000013276 |
| 3 | 7 688 382 | Alentejano pig | 0,5 | 0,5 | ENSSSCG00000031411 |
| 3 | 7 718 307 | Alentejano pig | 0,5 | 0,5 | ENSSSCG00000051021 |
| 3 | 7 719 451 | Alentejano pig | 0,5 | 0,5 | ENSSSCG00000007639 |
| 3 | 7 719 456 | Alentejano pig | 0,5 | 0,5 | ENSSSCG00000007639 |
| 3 | 41 851 708 | Alentejano pig | 0,5 | 0,5 | ENSSSCG00000049347 |
| 4 | 57 041 855 | Alentejano pig | 0,5 | 0,5 | ENSSSCG00000031362 |
| 4 | 81 354 492 | Alentejano pig | 0,5 | 0,5 | ENSSSCG00000006287 |
| 4 | 81 354 502 | Alentejano pig | 0,5 | 0,5 | ENSSSCG00000006287 |
| 4 | 81 460 964 | Alentejano pig | 0,5 | 0,5 | ENSSSCG00000006289 |
| 4 | 81 461 101 | Alentejano pig | 0,5 | 0,5 | ENSSSCG00000006289 |
| 4 | 103 296 739 | Alentejano pig | 0,5 | 0,5 | ENSSSCG00000006730 |
| 7 | 40 624 711 | Alentejano pig | 0,5 | 0,5 | ENSSSCG00000001710 |
| 8 | 5 293 | Alentejano pig | 0,5 | 0,5 | ENSSSCG00000047999 |
| 13 | 44 930 | Alentejano pig | 0,5 | 0,5 | ENSSSCG00000046931 |
| 14 | 113 396 910 | Alentejano pig | 0,5 | 0,5 | ENSSSCG00000010579 |
| 1 | 223 298 138 | Alentejano pig | 0,519 | 0,481 | ENSSSCG00000035668 |
| 1 | 245 476 692 | Alentejano pig | 0,519 | 0,481 | ENSSSCG00000005403 |
| 2 | 133 251 508 | Alentejano pig | 0,519 | 0,481 | ENSSSCG00000048403 |
| 3 | 7 719 528 | Alentejano pig | 0,519 | 0,481 | ENSSSCG00000007639 |
| 3 | 7 739 025 | Alentejano pig | 0,519 | 0,481 | ENSSSCG00000046 525 |
| 3 | 59 067 555 | Alentejano pig | 0,519 | 0,481 | ENSSSCG00000008228 |
| 4 | 54 771 671 | Alentejano pig | 0,519 | 0,481 | ENSSSCG00000051032 |
| 5 | 39 920 848 | Alentejano pig | 0,519 | 0,481 | ENSSSCG00000027503 |
| 5 | 40 053 513 | Alentejano pig | 0,519 | 0,481 | ENSSSCG00000027503 |
| 5 | 40 063 145 | Alentejano pig | 0,519 | 0,481 | ENSSSCG00000027503 |
| 6 | 77 051 136 | Alentejano pig | 0,519 | 0,481 | ENSSSCG00000023732 |
| 6 | 168 227 970 | Alentejano pig | 0,519 | 0,481 | ENSSSCG00000003960 |
| 8 | 5 386 | Alentejano pig | 0,519 | 0,481 | ENSSSCG00000047999 |
| 8 | 26 104 721 | Alentejano pig | 0,519 | 0,481 | ENSSSCG00000008767 |
| 13 | 177 038 091 | Alentejano pig | 0,519 | 0,481 | ENSSSCG00000048010 |
| 1 | 93 145 928 | Alentejano pig | 0,538 | 0,462 | ENSSSCG00000031966 |
| 1 | 129 366 035 | Alentejano pig | 0,538 | 0,462 | ENSSSCG00000037292 |
| 1 | 157 286 063 | Alentejano pig | 0,538 | 0,462 | ENSSSCG00000045840 |
| 3 | 7 685 456 | Alentejano pig | 0,538 | 0,462 | ENSSSCG00000031411 |
| 3 | 7 717 958 | Alentejano pig | 0,538 | 0,462 | ENSSSCG00000051021 |
| 3 | 7 718 387 | Alentejano pig | 0,538 | 0,462 | ENSSSCG00000051021 |
Determining the animal product's origin and breed based on the presence of at least one of said SNPs significantly associated with a specific animal breed.

2. Single Nucleotide Polymorphyms (SNPs) as defined in claim 1 for use as biomarkers for the identification of pig breeds.
